(19) Europäisches Patentamt
European Patent Office
Office européen des brevets

(11) Publication number: **0 180 398**
**A1**

(12) **EUROPEAN PATENT APPLICATION**

(21) Application number: 85307622.2

(22) Date of filing: 22.10.85

(51) Int. Cl.⁴: C 07 D 205/08

(30) Priority: 26.10.84 US 665372

(43) Date of publication of application: 07.05.86
Bulletin 86/19

(84) Designated Contracting States: AT BE CH DE FR GB IT LI LU NL SE

(71) Applicant: THE REGENTS OF THE UNIVERSITY OF CALIFORNIA, 2199 Addison Street, Berkeley California 95616 (US)

(72) Inventor: Overman, Larry E., 1300 Sand Castle Drive, Corona Del Mar California 92625 (US)
Inventor: Osawa, Tatsushi, Dept. of Chemistry 481 Physical Science Building, Univ. California Irvine California 92717 (US)

(74) Representative: Bannerman, David Gardner et al, Withers & Rogers 4 Dyer's Buildings Holborn, London, EC1N 2JT (GB)

(54) **Synthesis of beta-lactam.**

(57) A method of synthesizing monomeric formadelhyde imines in situ by reacting (cyanomethyl)- or (aminomethyl)-amines with organometal or Grignard reagents, and their use to synthesize 2-azetidinones (β-lactams) via reaction with enolates.

EP 0 180 398 A1

0180398

# SYNTHESIS OF BETA-LACTAMS

## FIELD OF THE INVENTION

This invention relates to a novel method of generating a wide spectrum of β-lactams particularly for medical uses.

## BACKGROUND OF THE INVENTION

As a result of the constant need for new and more efficacious antibiotics, partly due to the appearance of strains of bacteria that are genetically resistant to many commonly used antibiotics, considerable chemical research effort has been, and continues to be expended towards identifying novel naturally occurring antibiotics or developing synthetic methods of modifying them with the aim of creating drugs with enhanced antibiotic properties. Perhaps the class of antibiotics that has received the most attention in recent years is the 2-azetidinones, or as they are better known, β-lactams. β-lactams are cyclic amides of β-aminoacids, and hence are essentially nitrogen analogs of cyclic lactones. No doubt the search for new β-lactams is due to the fact that they are tolerated extremely well by humans, and that their molecular mechanism of action is known. β-lactams include penicillin, cephalosporin, and other widely used clinical antibiotics. The antibiotic activity of β-lactams is due to their ability to inhibit the synthesis of bacterial cell walls. Knowledge of the molecular biology of this event has enabled investigators to devise rational chemical synthetic schemes aimed at obtaining new β-lactams, and over the years a number of

synthetic schemes have yielded useful β-lactams as described in Chemistry and Biology of β-Lactam Antibiotics, Editors R. Morin and M. Gorman (Academic Press, New York, 1982). Recently considerable effort has focused on two subclasses of β-lactams, specifically those that are unsubstituted at the 4-position of the 2-azetidinone's heterocyclic ring. These are the nocardicins and monobactams. The former exhibit p-hydroxyphenylglycine and oxime functions, while the latter are 3-acylamino-2-oxoazetidine-1-sulfonic acids. Both classes exhibit broad spectrum antibiotic activity. Moreover, the nocardicins are unique in that they are especially active against Escherichia coli, Pseudomonas, and Proteus, while monobactams have the desirable feature of being resistant to β-lactamase, the bacterial enzyme responsible for hydrolyzing, and hence inactivating β-lactams. The heretofor unsuspected broad therapeutic uses of 4-unsubstituted β-lactams have generated research aimed at devising novel techniques for synthesizing 4-unsubstituted β-lactams, in addition to the nocardicins and monobactams. But there has not heretofore existed a broadly general, practical method of synthesizing a wide variety of 4-unsubstituted β-lactams in medicinally useful quantities.

Although there are a number of synthetic schemes for generating substituted β-lactams, they are not applicable to synthesizing 4-unsubstituted β-lactams. Generally used methods of synthesizing substituted β-lactams are: a) the condensation of ketenes and imines; b) cyclization of β-amino-acid derivatives via base catalysis; c) reaction of imines with acid chlorides in the presence of base; and,

d) a Reformatsky type reaction with imines, essentially a reaction of ester enolates with imines. Part of the reason for the unavailability of a general method to make 4-unsubstituted β-lactams has been the unavailability of monomeric formaldehyde imines with the formula $R-N=C<^{H}_{H}$ . While monomeric formaldehyde imines are known to exist in the gas phase, in solution they rapidly trimerize to hexahydro-1,3,5-triazines with the formula

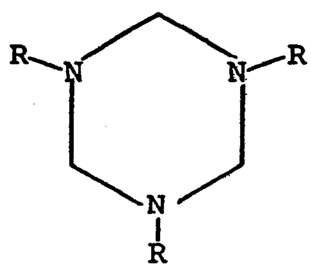

Thus, the use of imines in β-lactam synthesis has been restricted to using stable aldimines with the formula $R-N=C<^{R^1}_{H}$ as described by Gilman and Specter in the Journal of the American Chemical Society, 1943, Vol. 65, p. 2255, and Gluchowski et al in the Journal of Organic Chemistry, 1980, Vol. 45, p. 3413. These reagents owe their stability to the presence of the R' group which prevents trimerization, but at the same time bars unsubstitution at the 4- position of the β-lactam ring since it is incorporated at that position.

Because of the unavailability of techniques to generate monomeric formaldehyde imines, few 4-unsubstituted β-lactams are produced synthetically. Those that have been are generated either by tedious and complex chemical

reactions which require removal of a group at the 4 position after formation of the β-lactam, or by an acid catalyzed reaction that involves the use of hexahydrotriazines, that is, the trimerized product of formaldehyde imines. The latter method, as described by Kamiya et al in Tetrahedron Letters, 1978, p. 5119, and more recently by Akeda et al, in the Chemistry Pharmacology Bulletin, 1981, Vol. 29, p. 1747, is useful for generating nocardicins, but has not been used as a general means of generating other types of 4-unsubstituted β-lactams.

It is clear in light of the need for novel antibiotics, and the fact that there currently does not exist a synthetic scheme for generating a wide variety of 4-unsubstituted β-lactams, that a method for producing these antibiotics would be a valuable tool of the medicinal chemist.

**SUMMARY OF THE INVENTION**

According to this invention a simple one step method is provided which permits the synthesis of a diverse array of medically useful 4-unsubstituted 2-azetidinones premised on the *in situ* formation of monomeric formaldehyde imines from secondary N-(cyanomethyl) or N-(aminomethyl) amines, and reacting the formaldehyde imines with metal ester enolates to form the 4-unsubstituted 2-azetidinones. The method allows for the efficient production of both known and previously difficult to make antibiotics, and hithertofore unknown optically active 2-azetidinones.

**BRIEF DESCRIPTION OF THE DRAWINGS**

Table I. General scheme for making 4-unsubstituted β-lactams, and their per cent yields obtained.

<GREGHPA.H13>

Table II. Alternate scheme for making 3-amino-substituted β-lactams, and their per cent yields obtained.

Table III. Synthetic scheme for 3-N-(acylamino)-4-unsubstituted β-lactams, and their per cent yields.

Table IV. Synthesis of 3 representative nonracemic 4-unsubstituted β-lactams.

**DETAILED DESCRIPTION OF THE INVENTION**

This invention was made with Government support under Contract Grant No. GM 30859 awarded by the National Institute of Health and Grant No. CHE 8203336 awarded by the National Science Foundation. The Government has certain rights in this invention.

General Procedure

The general method of synthesizing 4-unsubstituted β-lactams is illustrated as follows. Two equivalents of a starting ester are allowed to react with any base capable of forming the metal ester enolate, particularly lithium hexamethyldisilazide (LHMDS) or lithium diisopropylamine (LDA). The reaction is performed between -80°C and 0°C, the preferred temperature being -70°C, and in an organic solvent particularly tetrahydrofuran, diethyl ether, dioxane, or dimethoxyethane. Next, a secondary N-(cyanomethyl)amine or N-(aminomethyl)amine, formed from primary amines by the commonly used methods of either Baker et al, as described in the Journal of the Chemical Society, 1949, p.307, or Okawara and Harada as described in the Journal of Organic Chemistry, Vol. 37, p. 3286, 1972, is added, at a temperature between -80°C and 25°C, the preferred temperature being -60°C. This step results in the *in situ* generation of the corresponding formaldehyde imine as described by Overman and Burk in

Tetrahedron Letters, Vol. 25, p. 1635, 1984, and the subsequent trapping of this intermediate by the metal ester enolate. The reaction mixture is then allowed to warm to -20°C and room temperature and after several hours is extracted with an organic solvent, washed, dried and concentrated. Chromatography yields the 2-azetidinone.

Using the reaction conditions described above, 3-amino-substituted 4-unsubstituted β-lactams having a hydrogen substituent at C-3 of the β-lactam ring can be made from a metal ester enolate of a silyl-protected glycine ester. The latter is formed by Djuric's method as described in Tetrahedron Letters, 1981, Vol. 22, p. 1787, wherein the disilyl reagent 1, 1, 4, 4-tetramethyl-1,4-dichlorodisilethylene is reacted with a glycine ester in dichloromethane in the presence of triethylamine. Chromatographic purification on silica gel results in cleavage of the silyl protecting group to give directly the 3-amino-2-azetidinone product.

3-N-(acylamino)-4-unsubstituted β-lactams can be prepared similarly by employing metal dianions of N-acyl alpha-amino esters. Although a number of bases, as described by Bartlett and Barstow in the Journal of Organic Chemistry, Vol. 47, p. 3933, 1982, can be employed for dianion generation, the preferred base is lithium hexamethyldisilazide.

Nonracemic 4-unsubstituted β-lactams can be generated from N-(cyanomethyl)amines derived from enantiomerically pure primary amines, and using the general reaction conditions described above. Reaction of a (R)-cyanoamine with about 2 equivalents of a metal ester enolate yields the

(R), (R)- β-lactam in better than 50% yields and high diastereoselectivity. β-lactams with the "natural" 3S configuration can be prepared similarly from (S)-cyanoamines.

The following examples, while exemplary of the present invention, should not be construed as specifically limiting the invention, and such variations which would be within the purview of one skilled in this art are to be considered to fall within the scope of this invention.

EXAMPLE I - General Procedure

Synthesis of 1-benzyl-3,3-dimethyl-2-azetidinone.

0.81 mL of diisopropylamine equivalent to 5.80 millimoles and 20 mL of tetrahydrofuran was cooled to -70°C and then 2.4 mL of a 2.2 molar solution of n-butyllithium in hexane equivalent to 5.29 millimoles was added. This solution was stirred for 30 minutes at -70°C and then a solution of ethyl isobutyrate containing 614 mg (5.3 millimoles) and 5 mL of tetrahydrofuran was added dropwise. The reaction solution was stirred for 1 hour at -70°C and then a solution of (benzylamino)acetonitrile containing 370 mg of the nitrile (2.53 millimoles), and 5 mL of tetrahydrofuran was added dropwise. After 1 hour at -70°C the reaction mixture was allowed to warm up to room temperature, and after 20 hours was quenched by adding excess solid ammonium chloride and water. The quenched reaction mixture was extracted with $CHCl_3$, and the organic extract was washed with brine, dried with sodium sulfate ($Na_2SO_4$), and concentrated. Flash chromatography utilizing silica gel, in a mixture of hexane-acetone (96:4) yields 317 mg (66%) of the 2-azetidinone as a colorless oil with

the properties: $^1H$ NMR (250 MHz, $CDCl_3$) 7.20-7.40 (m, $NCH_2$<u>Ph</u>), 4.34 (s, $NC\underline{H}_2Ph$), 2.91 (s, C-4 $CH_2$), 1.30 (s, C-3 Me); $^{13}C$ NMR (63 MHz, $CDCl_3$) 174.1, 136.2, 129.0, 128.3, 127.8, 54.1, 51.1, 45.8, 21.3; IR ($CHCL_3$) 1738 $cm^{-1}$; MS (CI) m/e 190 (MH).

Table I shows the general procedure for making 4-unsubstituted β-lactams and those that have been made and their per cent yields.

EXAMPLE II

<u>Synthesis of 3-amino-1-(2,4-dimethoxybenzyl)-2-azetidinone.</u>

4.96 millimoles of n-butyllithium, 1.98 mL of a 2.5 molar solution in hexane was added to a solution containing hexamethyldisilazane (1.15 mL, 5.48 millimoles) and 24 mL of tetrahydrofuran at -70°C. This reaction mixture was stirred for 30 minutes and then a solution of ethyl 2-(2,2,4,4-tetramethyl-2,5-disilylazacyclopentyl)-glycinate containing 1.21 gm of the glycinate or 5.0 millimoles, and 6 mL of tetrahydrofuran was added dropwise. The reaction mixture was allowed to stir for another hour at -70°C and then the solution containing 486 mg or 2.36 millimoles of [(2,4-dimethoxybenzyl)amino]acetonitrile and 6 mL of tetrahydrofuran was added dropwise. This solution was stirred at -70°C for 2 hours and then at room temperature for 20 hours and the reaction terminated with solid $NH_4Cl$ and water. The mixture was next poured into ice water and the product isolated with $CHCl_3$. The $CHCl_3$ extract was washed with brine, dried with sodium sulfate ($Na_2SO_4$) and concentrated. The residue is purified by flash chromatography using silica gel and $CHCl_3$/MeOH (99:1) to give 449 mg (81%) of the 2-azetidinone product as a pale

yellow oil with the properties: $^{1}H$ NMR (250 MHz, $CDCl_3$) 7.11 (d, J= 8.9 Hz, C-6 ArH), 6.46 (d, J = 2.5 Hz, C-3 ArH), 6.44 (dd, J = 8.9, 2.5 Hz, C-5 ArH), 4.31 (app br s, $NCH_2Ph$), 4.10 (m, C-3 H), 3.80 (s, OMe) 3.81 (s, OMe), 3.40 (app t, J = 5.3 Hz, C-4 H), 2.87 (dd, J = 5.3, 2.2 Hz; C-4 H), 1.72 (br s, $NH_2$); IR ($CHCl_3$) 1747 $cm^{-1}$; MS (CI) m/e 237 (MH), 193, 151.

Table II shows this scheme for making 3-amino-substituted β-lactams and some of the molecules made and their per cent yields.

EXAMPLE III

Synthesis of 3-N-(acylamino)-4-unsubstituted β-lactams: 3-[(benzyloxycarbonyl)amino]-1-(2,4-dimethosybenzyl)2-azetidinone

A solution of $(Me_3Si)_2NLi$, prepared from $(Me_3Si)_2NH$, 0.6 mL or 2.74 millimoles, and n-butyllithium (0.99 mL of a 2.5 molar solution in hexane, 2.5 millimoles) in 12 mL of tetrahydrofuran was added at -70°C to a solution containing 294 mg or 1.24 millimoles of ethyl N-(benzyloxycarbonyl)glycinate and 3 mL of tetrahydrofuran. The reaction was allowed to proceed for one hour at -70°C and then a solution containing 122 mg or 0.59 millimoles of [(2,4-dimethoxybenzyl)amino]acetonitrile and 3 mL of tetrahydrofuran was added at -70°C and the resulting solution stirred at this temperature for 1 hour and then the temperature allowed to increase to between -20 to -25°C, and stirred for another 3 hours. The reaction was neutralized with acetic acid in tetrahydrofuran, and the mixture poured into ice water and extracted with $CHCl_3$. The extract was washed with brine, dried with sodium sulfate ($Na_2SO_4$),

<GREGHPA.H13>

concentrated, and purified by flash chromatography utilizing silica gel and $CHCl_3$ to yield 138 mg (63%) of the product to 2-azetidinone as a colorless solid. Recrystallization from ethyl acetate-hexane gave an analytical sample of fine needles with the properties: mp 102-104°C; $^1H$ NMR ($CDCl_3$) 7.33 (br s, $OCH_2\underline{Ph}$), 7.12 (br d, J = 8.9 Hz, C-6 Ar$\underline{H}$), 6.43 (m, 2H, Ar$\underline{H}$), 5.62 (m,NH), 5.09 (s, $C\underline{H}_2Ph$), 4.79 (m,C-3 H), 4.33 (m, $NC\underline{H}_2Ph$), 3.80 (s, two OMe), 4.43 (m, C-4 H), 3.08 (m, C-4 H); IR ($CHCl_3$) 3443, 1760, 1727 $cm^{-1}$; MS (CI) m/e 371 (MH), 263, 261, 151.

Table III shows the synthetic scheme for 3-N-(acylamino)-4-unsubstituted β-lactams, some of the molecules produced and their per cent yields.

EXAMPLE IV

<u>Synthesis of nonracemic 4-unsubstituted β-lactams: 3(R)-amino-1-[2-benzyloxy-1(R)-(4-benzyloxy)phenylethyl]-2-azetidinone</u>

A solution of ethyl 2-(2, 2, 4, 4 - tetramethyl - 2, 5 -disilylazacyclopentyl)glycinate containing 607 mg or 2.40 millimoles of the glycinate and 3 mL of tetrahydrofuran was treated at -70°C with $(Me_3Si)_2NLi$, prepared from $(Me_3Si)_2NH$(0.58 mL or 2.76 millimoles) and n-butyllithium (0.99 mL of a 2.5 molar solution in hexane, or 2.5 millimoles) in 12 mL of tetrahydrofuran at -70°C. The reaction was allowed to proceed for 1 hour at -70°C and then a solution containing 440 mg or 1.18 millimoles of [(2-benzyloxy)-1(R)-(4-benzyloxy)phenyl)ethyl]-aminoacetonitrile and 3mL of tetrahydrofuran was added and the resulting solution stirred at -70°C for 2 hours and then at room temperature for 20 hours. The reaction was terminated with

<GREGHPA.H13>

solid $NH_4Cl$ and extracted with $CHCl_3$. The organic extract was then washed with brine, dried with sodium sulfate ($Na_2SO_4$), concentrated and the residue purified by flash chromatography using silica gel and $CHCl_3$/MeOH(98:2) to yield 342 mg (72%) of the azetidinone as a pale yellow oil. The 250 MHz, $^1$NMR spectrum revealed that the sample was contaminated with only 9% of the C-3 diastereoisomer. The pure 3(R)-2-azetidinone has the properties: $^1$H NMR (250 MHz, $CDCl_3$) 7.17-7.42 (m, 12H, ArH), 6.19-6.96 (m, 2H, ArH), 5.03 (s, $OCH_2Ph$), 4.89 (dd, J = 5.0, 8.5 Hz, NCHArH), 4.57 (AB quartet, $OCH_2Ph$), 4.08 (dd, J = 2.3, 5.4 Hz, $CHNH_2$), 3.95 (dd, J=10.1, 8.5 Hz, CHHOBn), 3.76 (dd, J=10.1, 5.0 Hz, CHHOBn), 3.45 (app t, J = 5.5 Hz, <u>trans</u> C-4 H), 3.06 (dd, J = 2.3, 5.5 Hz, <u>cis</u> C-4 H), 1.92 (br s, $NH_2$); IR ($CHCl_3$) 1761 $cm^{-1}$; MS(CI) m/e 403(MH), 375; $[\alpha]_D^{25}$ -31.6°(c 1.05, $CHCl_3$),>90% e.e.

Table IV shows the synthesis of other representative nonracemic 4-unsubstituted β-lactams.

<GREGHPA.H13>

## CLAIMS

1. A method for synthesizing β-lactams comprising the steps of generating monomeric formaldehyde imines from secondary amines by reaction with a strong base in solution; and

reacting said formaldehyde imines with ester enolates.

2. A method as defined in Claim 1 in which said secondary amines have the formula $XCH_2NHR^1$ and form 4-unsubstituted β-lactams with the structure where X

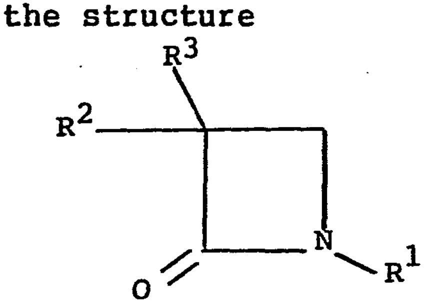

is a good leaving group, and $R^1$ is any alkyl, aryl, or cyclic group and $R^2$ and $R^3$ are hydrogen, an amino group, alkoxy group, thioalkoxy group, or any alkyl, n-acyl, aryl, or cyclic group.

3. A method as defined in Claim 1 in which the strong base is a metal ester enolate.

4. A method as defined in Claim 3 in which the metal ester enolate is generated by reaction of an ester with lithium hexa-methyldisilazide or lithium diisopropylamide.

<GREGHPA.H13>

5. A method as defined in Claim 2 in which X is either a CN or an amino group.

6. A method as defined in Claim 1 in which formaldehyde imine generation and reaction with the metal ester enolate is initially carried out in the temperature range of -78°C to 0°C and subsequently carried out at room temperature.

7. A method as described in Claim 1 in which the ester is replaced by a carboxylic acid derivation having the formula $R^2R^3CHCO\text{-}X^1$ and $X^1$ is a good leaving group.

8. A method as defined in Claim 7 wherein a good leaving group is an alkoxy, acyloxy, imide or thio group.

9. A method as defined in Claim 1 wherein the ester has the structure

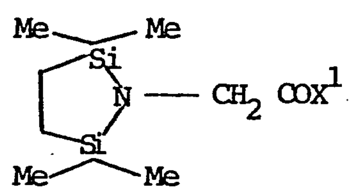

10. A method as defined in Claim 1 wherein the ester has the structure 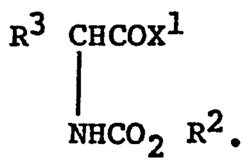

11. A method as defined in Claims 7, 9 and 10 in which the ester or carboxylic acid derivative is reacted with a base to form an enolate.

<GREGHPA.H13>

12. A method as defined in Claim 10 wherein the base is lithium diisopropylamide or lithium hexamethyldisilazide.

13. A method as defined in Claim 2 in which 4-unsubstituted β-lactams produced are optically active by reacting enantiomerically pure secondary amines prepared from enantiomerically pure primary amines with ester enolates.

<GREGHPA.H13>

## TABLE I

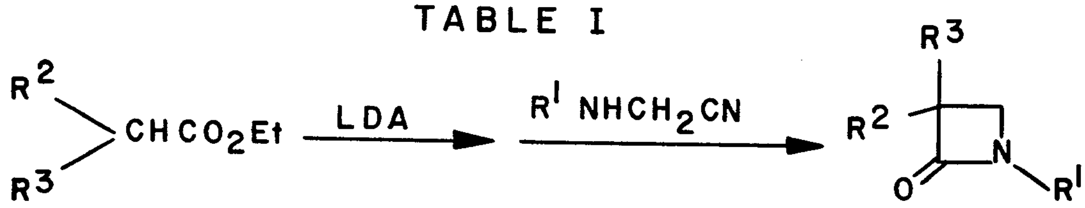


| | % YIELD |
|---|---|
| 1. $R^1 = CH_2Ph$, $R^2 = R^3 = Me$ | 66% |
| 2. $R^1 = Ph$, $R^2 = R^3 = Me$ | 77% |
| 3. $R^1 =$ 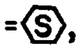, $R^2 = R^3 = Me$ | 66% |
| 4. $R^1 = CH_2Ph$; $R^2, R^3 = -(CH_2)_5$ | 65% |
| 5. $R^1 = CH_2Ph$, $R^2 = Me$, $R^3 = SPh$ | 62% |

## TABLE II

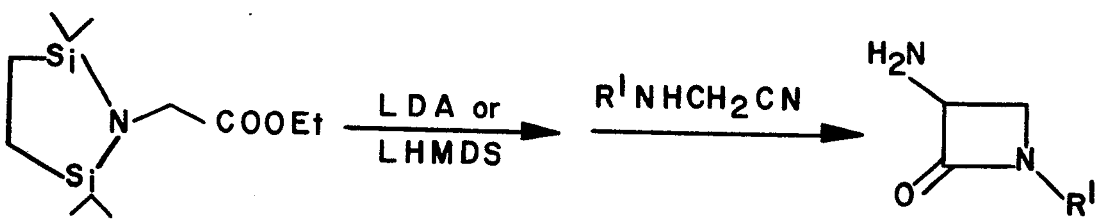


| | % YIELD |
|---|---|
| 1. $R^1 = CH_2Ph$ | 43% |
| 2. $R^1 = CH_2$ 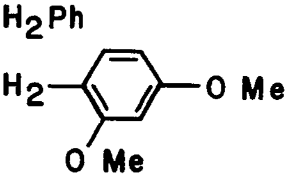 | 81% |
| 3. $R^1 =$ 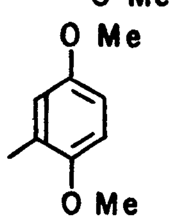 | 47% |

TABLE III

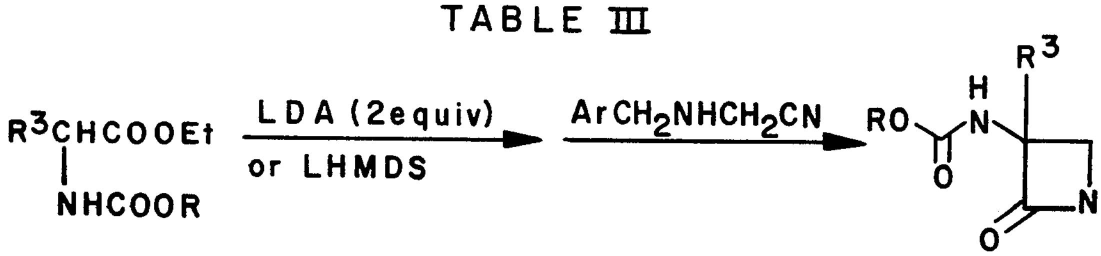


| | | % YIELD |
|---|---|---|
| 1. Ar = 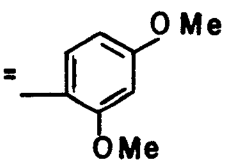 | ; R = $CH_2Ph$, $R^3$ = Me | 74 % |
| 2. Ar = 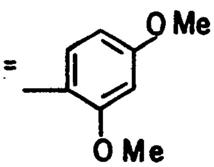 | ; R = $Bu^t$, $R^3$ = Me | 60% |
| 3. Ar = 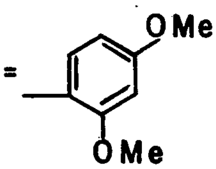 | ; R = $Bu^t$, $R^3$ = $CHMe_2$ | 45 % |
| 4. Ar = 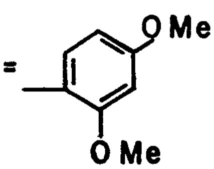 | ; R = $CH_2Ph$, $R^3$ = H | 63 % |
| 5. Ar = Ph | ; R = $CH_2Ph$, $R^3$ = Me | 24 % |
| 6. Ar = Ph | ; R = $Bu^t$, $R^3$ = Me | 41% |

# TABLE IV

1.

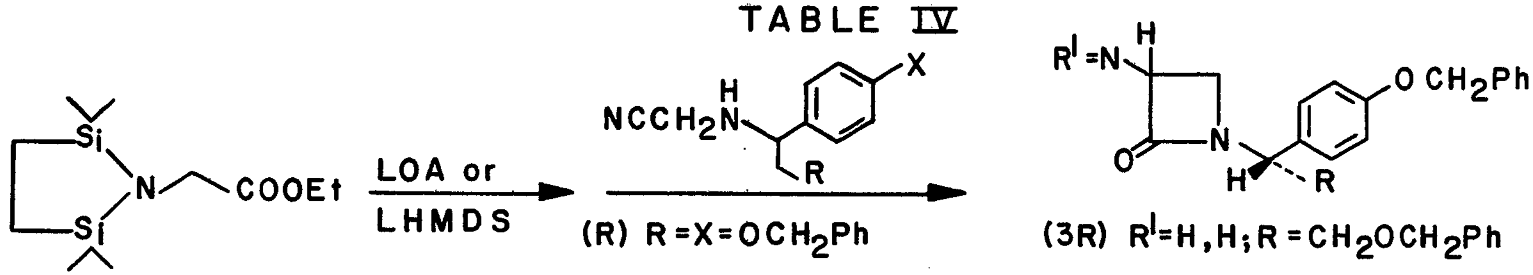


(R) R = X = $OCH_2Ph$

(3R) $R^1$ = H, H; R = $CH_2OCH_2Ph$

2.

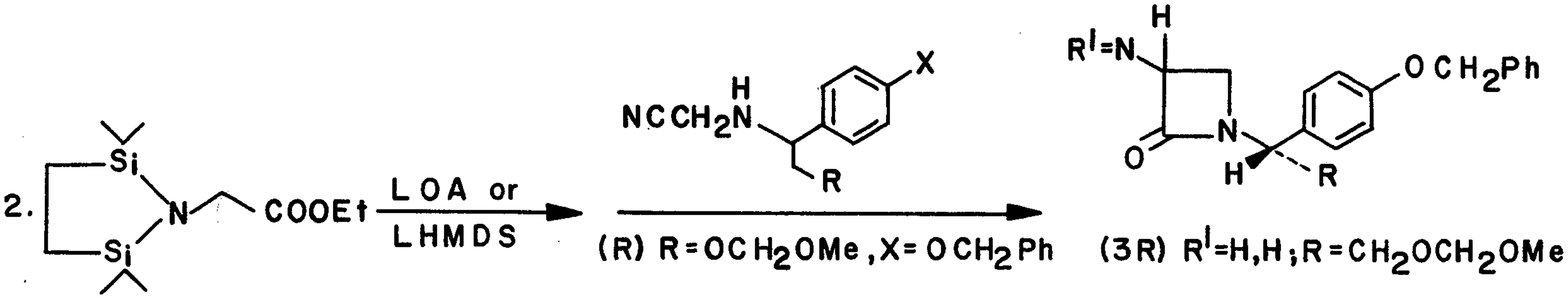


(R) R = $OCH_2OMe$, X = $OCH_2Ph$

(3R) $R^1$ = H, H; R = $CH_2OCH_2OMe$

3.

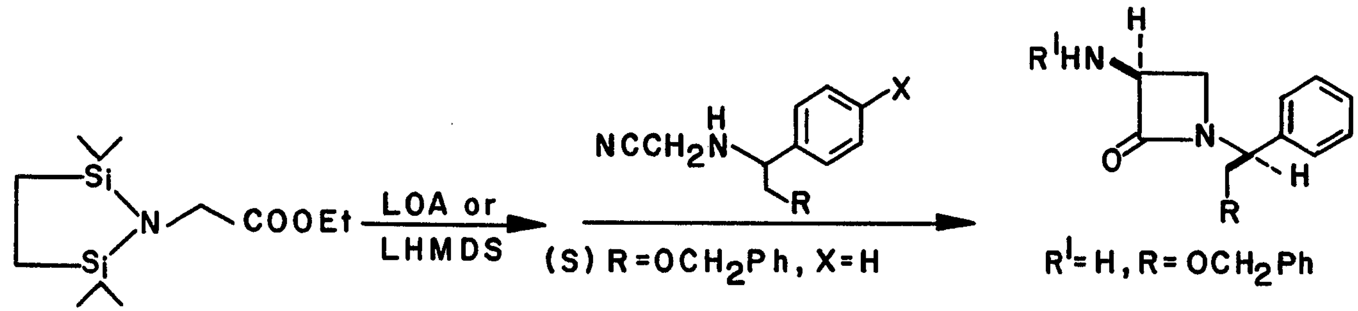


(S) R = $OCH_2Ph$, X = H

$R^1$ = H, R = $OCH_2Ph$

0180398

European Patent Office

# EUROPEAN SEARCH REPORT

Application number

EP 85 30 7622

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.4) |
|---|---|---|---|
| Y | TETRAHEDRON LETTERS, vol. 25, no. 20, 1984, pages 2143-2146, Pergamon Press Ltd., GB; J.-E. DUBOIS et al.: "Ketene bis(trimethylsilyl) acetals. Cross-aldol type condensation reactions with aldehydes and schiff bases"<br>* Whole document *<br>--- | 1,2 | C 07 D 205/08 |
| Y | JOURNAL OF THE AMERICAN CHEMICAL SOCIETY, vol. 106, no. 17, 22nd August 1984, pages 4819-4825, American Chemical Society, US; D.-C. HA et al.: "N-Trimethylsilyl Imines: Applications to the Synthesis of beta-Lactams"<br>* Whole document *<br>--- | 1,2 | TECHNICAL FIELDS SEARCHED (Int. Cl.4) |
| Y | US-A-4 181 800 (T. KAMIYA)<br>* Columns 3,4, processes 1,2 *<br>--- | 1,2 | C 07 D 205/00 |
| D,Y | JOURNAL OF ORGANIC CHEMISTRY, vol. 45, no. 17, 15th August 1980, pages 3413-3416, American Chemical Society, US; C. GLUCHOWSKI et al.: "Preparation of beta-lactams by the condensation fo lithium ester enolates with aryl aldimines"<br>* Whole document *<br>--- -/- | 1,2 | |

The present search report has been drawn up for all claims

| Place of search | Date of completion of the search | Examiner |
|---|---|---|
| THE HAGUE | 29-01-1986 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82

0180398

European Patent Office

# EUROPEAN SEARCH REPORT

Application number

EP 85 30 7622

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | JOURNAL OF THE AMERICAN CHEMICAL SOCIETY, vol. 107, no. 6, 20th March 1985, pages 1698-1702, American Chemical Society, US; L.E. OVERMAN et al.: "A convenient synthesis of 4-unsubstituted beta-lactams" * Whole document * ----- | 1-13 | |

TECHNICAL FIELDS SEARCHED (Int. Cl.4)

The present search report has been drawn up for all claims

| Place of search | Date of completion of the search | Examiner |
|---|---|---|
| THE HAGUE | 29-01-1986 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document